# EUROPEAN PATENT APPLICATION

(11) **EP 4 732 778 A1**
(43) Date of publication of application: **29.04.2026**
(21) Application number: 24209280.7
(22) Date of filing: 28.10.2024
(51) Int. Cl.: A61B 6/50, A61B 6/00

(54) **DEVICES AND METHODS FOR DETERMINING AN IMAGING DIRECTION FOR ROBUSTLY DETECTING AND CLASSIFYING BONE FRACTURES IN A 2D PROJECTION IMAGE**

(71) Applicant: metamorphosis GmbH, 33100 Paderborn (DE)
(72) Inventor: BLAU, Arno, 42336 Balingen (DE)
(74) Representative: LKGLOBAL Partnerschaftsgesellschaft mbB

(57) **Abstract**

Device and Method for assisting in fracture diagnosis are provided. The device comprises a processing unit and a computer program product, when executed on the processing unit, causes the device to receive image data, to interpret the image data, and to provide an output based on the image data interpretation. The received image data represents an anatomical region including an injured part of a human body. It may be emphasized that the image data may include data of a single X-ray projection image. Interpreting the image data includes estimating the likelihood of a fracture, wherein, when the likelihood is below a predetermined value, the provided output includes information of an imaging direction allowing a further assessment of the injured part of the human body, and wherein, when the likelihood is above the predetermined value, the provided output includes information of an imaging direction allowing a confirmation of the fracture.

## Description

### FIELD OF INVENTION

The invention relates to diagnostics, robotic assisted diagnostics, as well as to computer and robotic-assisted surgery.

The devices and methods described herein can be applied to assist in performing steps in bone fracture diagnostics or musculoskeletal surgery.

### BACKGROUND OF THE INVENTION

Finding and classifying bone fractures is often not an easy task. Many fractures are not easy depicted or detected in simple 2D X-ray images. There is often the need for a certain imaging direction in order to depict thin fracture lines and/ or minimal offsets of bone fragments. Achieving these optimal imaging directions often is a real art and need highly skilled radiation technicians. So, in many cases when in doubt a CT is made instead which obviously comes with a high radiation exposure to the patient. So, the choice is often not to acquire a CT scan so that many fractures are not diagnosed at all and stay either untreated or treated wrongly.

### SUMMARY OF THE INVENTION

The mentioned problems are mitigated or solved by the subject matter according to each of the independent claims. Further embodiments are described in the respective dependent claims.

It is intended to recognize aspects of fractures and to detect if the current viewing direction of an imaging device relative to a patient is more or less optimal for detecting an assumed fracture.

In general, a device for assisting in fracture diagnosis is provided, the device comprising a processing unit and a computer program product. The computer program product when executed on the processing unit causes the device to receive image data, to interpret the image data, and to provide an output based on the image data interpretation. The received image data represents an anatomical region including an injured part of a human body. It may be emphasized that the image data may include data of a single X-ray projection image.

Interpreting the image data includes estimating the likelihood of a fracture, wherein, when the likelihood is below a predetermined value, the provided output includes information of an imaging direction allowing a further assessment of the injured part of the human body, and wherein, when the likelihood is above the predetermined value, the provided output includes information of an imaging direction allowing a confirmation of the fracture.

The value of the likelihood of a fracture may be determined taking into account the actually injured part of the human body and/or the circumstances of the injury. For example, having an accident with a bicycle will cause injuries typical for such an accident. That is, the information about those circumstances may be provided to the device by way of an input and the device will, in consequence, assume a predetermined value for the likelihood of a fracture. In a case with a higher likelihood of a fracture, the device may provide an output including information of a suitable imaging direction even before generating the first X-ray image.

According to an embodiment, the device may be caused, when the likelihood is above the predetermined value, to classify the fracture as a specific fracture out of a plurality of possible fractures. In consequence, an output may be provided including information of an imaging direction allowing a confirmation of the classification of the fracture. It will be understood that no further image may be necessary in case the classification of the fracture can be determined with sufficient certainty on the basis of the one, e.g., the first X-ray image.

According to an embodiment, the interpretation of the image data is based on a neural network being trained based on 3D image data of different fractures of bones and on 2D projections from different directions showing those fractures. The neural network may be trained with a plurality of 3D imaging data of fractures bones. The training may include segmenting important edges of the image, like outline of the bone in the 2D image, certain inner edges, and certain inner edges that would be outline of the bone from another imaging direction. The neural network may alternatively or additionally be trained with a plurality of 2D images depicting fractured bones. Further, the neural network may be trained with a plurality of 2D images from different imaging directions of the same situation of a fractured bone, for a plurality of fractured bones. Moreover, varying image quality may be applied for all mentioned training procedures to enhance robustness of performance during inference.

According to a further embodiment, the device may be caused to determine the imaging direction of the X-ray projection image, and, when the likelihood is above the predetermined value, the output may include information regarding a deviation between the determined imaging direction and the imaging direction allowing the confirmation of the fracture. The information regarding the deviation may be provided to a robotic X-ray imaging device causing the same to generate a new X-ray image from the imaging direction allowing the confirmation of the fracture.

As a further aspect, the device may be caused to assess the feasibility of generating the new X-ray image taking into account movement restrictions for the X-ray imaging device. For example, it is not always possible to generate an X-ray image with a preferred imaging direction, because other parts of the human body may hinder the positioning of the X-ray source and detector relative to the human body for imaging the injured part of the human body.

As discussed above, the value of the likelihood of a fracture may depend on the actual injury. In consequence, the value of the likelihood may be between 0,5 and 0,9, preferably between 0,6 and 0,8. The value may cover a range or may be a specific value like a threshold. For example, the likelihood value may be 0.5, 0.6, 0.7, 0.8, or 0.9, or any other specific value. In other words, it is less likely that a fracture can be recognized in a first image, and it is more likely that either a fracture may be assumed or no indication of a fracture can be found in the first image. According to the disclosure, a specific imaging direction is suggested in case a fracture has been detected, or a typical, more general imaging direction is suggested in case of uncertainty about a fracture in the X-ray image.

According to another aspect of the disclosure, a method of assisting in fracture diagnosis is provided, the method comprising the steps of receiving image data, interpreting the image data, and providing an output based on the image data interpretation. The received image data represents an anatomical region including an injured part of a human body and the image data include data of a single X-ray projection image. Interpreting the image data includes estimating the likelihood of a fracture, with all features as described above. When the likelihood is below a predetermined value, an output is provided including information of an imaging direction allowing a further assessment of the injured part of the human body, and when the likelihood is above the predetermined value, an output is provided including information of an imaging direction allowing a confirmation of the fracture.

Furthermore, the fracture may be classified as a specific fracture out of a plurality of possible fractures. If there is any uncertainty regarding the classification of the fracture, an output may be provided including information of an imaging direction allowing a confirmation of the classification of the fracture.

While a neural network may be utilized when interpreting the image data of an X-ray image, the output may be provided to a robotic X-ray imaging device causing the same to generate a new X-ray image from the imaging direction allowing the confirmation of the fracture.

An algorithm or deep neural net may be able to determine the kind of fractures or depicted anatomical regions of the image for which the imaging direction is already suitable (e.g. it may be able to show a confident negative result with regards to this anatomical region and/or fracture types. It may be able to provide confidence values to exclude the availability of certain fracture types and/or for certain anatomical regions. It may also provide information on regions (e.g depicted or adjacent to depicted areas where a fracture cannot be included). It can provide guidance on how to reposition the imaging device in order to have sufficient imaging information to either exclude or confirm a fracture line or certain fracture types of certain anatomical areas, that may already be depicted but not from a sufficient viewing angle or that are adjacent to the imaging region. One or more neural networks may be trained with real and/or simulated imaging data on the appearance of fracture lines or offsets of bone fragments.

Moreover, an algorithm may be able to calculate a 3D reconstruction of a fractured bone based on detecting the outlines of the bone in a 2D projection image and on a detection of at least one fracture line. Another algorithm may be able to render a virtually reconstructed 2D projection (e.g. a virtual X-ray image) from any desired imaging direction. Based on above calculated 3D reconstruction and a rendering of at least one virtual 2D projection image with another imaging direction than that of the real 2D projection image, an algorithm decides if a fracture may be better visible in the image and if so, requests a new real projection image from this imaging direction. The algorithm may decide based on interpretation of a plurality of virtually calculated images which imaging out of the plurality would be suitable. Another algorithm or a neural network may interfere with this decision based on feasibility of acquisition of this imaging direction (not all may be possible or easy enough to acquire). The requested image from another imaging direction may be obtained manually or by a robotic imaging device.

Finally, a computer program product may be provided as a further aspect of the disclosure, including instructions, when executed on a processing unit of a device as described above, causes the device to perform the described method. The computer program product may preferably be loaded into the random-access memory of a data processor. The data processor or processing unit of a device according to an embodiment may thus be equipped to carry out at least a part of the described method or process. Further, the disclosure may relate to a computer-readable medium such as a CD-ROM on which the disclosed computer program product may be stored. However, the computer program product may also be presented over a network like the World Wide Web and can be downloaded into the random-access memory of the data processor from such a network. Furthermore, the computer program product may also be executed on a cloud-based processor, with results presented over the network.

As should be clear from the above description, a main aspect is a processing of X-ray image data, allowing an automatic interpretation of visible objects. The methods described herein are to be understood as methods assisting in a surgical treatment of a patient. Consequently, the method may not include any step of treatment of an animal or human body by surgery, in accordance with an embodiment.

It is further noted that embodiments are described with reference to different subject-matters. In particular, some embodiments are described with reference to method-type claims (computer program product) whereas other embodiments are described with reference to apparatus-type claims (system/device). However, a person skilled in the art will gather from the above and the following description that, unless otherwise specified, any combination of features belonging to one type of subject-matter as well as any combination between features relating to different subject-matters is considered to be disclosed with this application.

The aspects defined above and further aspects, features and advantages of the present invention can also be derived from the examples of the embodiments to be described hereinafter and are explained with reference to examples of embodiments also shown in the figures, but to which the invention is not limited.

### BRIEF DESCRIPTION OF THE DRAWINGS

Exemplary aspects of the disclosure will be described in the following with reference to the following figures:
Fig. 1 shows an X-ray image of a right hand with slightly bended fingers.
Fig. 2 shows an X-ray image of a right hand from above.
Fig. 3 shows an X-ray image of a right hand from above generated with different imaging parameters.
Fig. 4 shows an X-ray image of a right hand from a side.
Fig. 5 is a flow chart illustrating steps of a method in accordance with the disclosure.

### DETAILED DESCRIPTION OF EMBODIMENTS

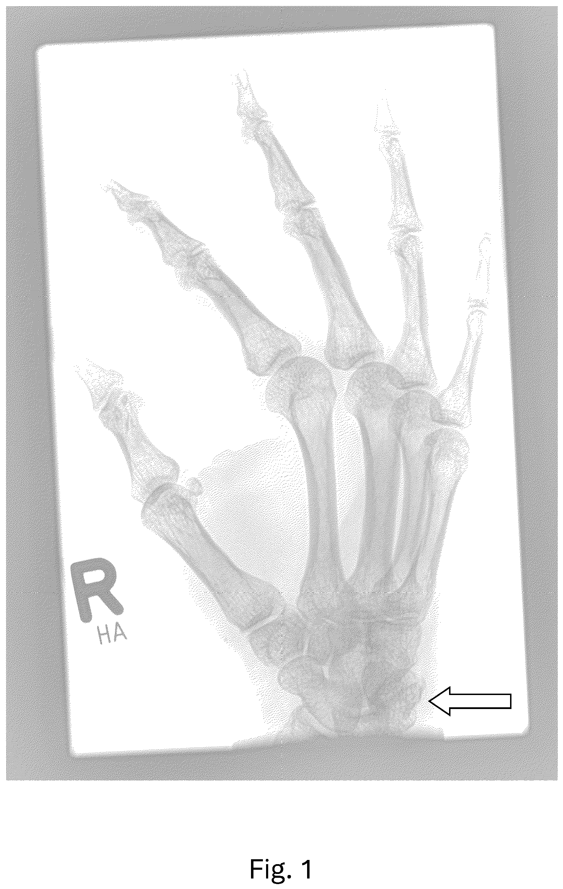

Figures 1 to 4 illustrate the difficulty of detecting and classifying a fracture.

Figure 1 is an X-ray image of a right hand having a fracture at the hand j oint. As indicated by the white arrow, the fracture is assumed to be on the outer side of the hand joint. However, it is difficult to see whether the fracture is at the pisiform bone or at the triquetrum bone because these bones are in front and behind each other. Further, it is difficult to detect the fracture due to the irregular bone edges.

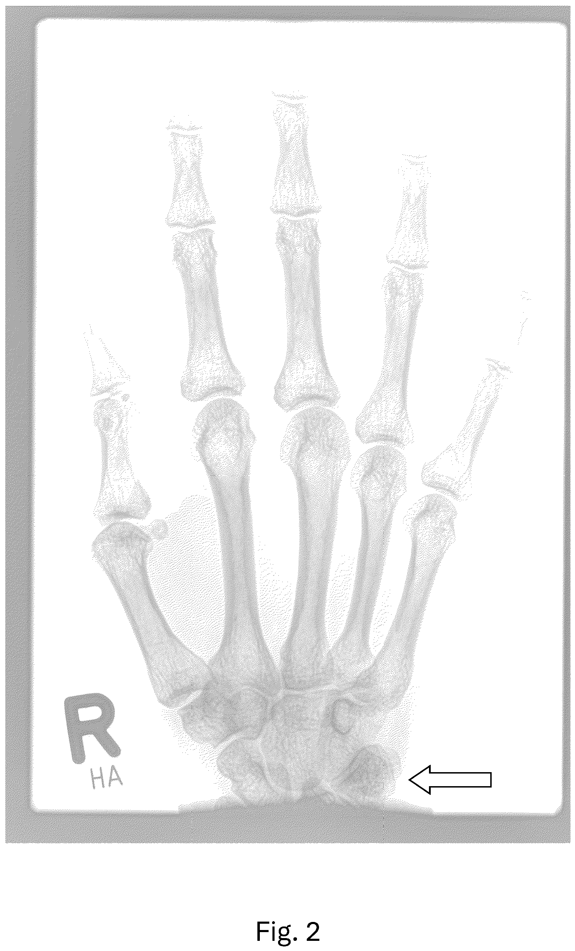

Figure 2 is another X-ray image of a right hand showing a top view onto the hand lying flat. Here again, a white arrow indicates the assumed location of a fracture at the hand joint. For the same reasons as in figure 1, it is difficult to recognize the fracture in figure 2, and particularly to determine a relation of the fracture to a specific bone.

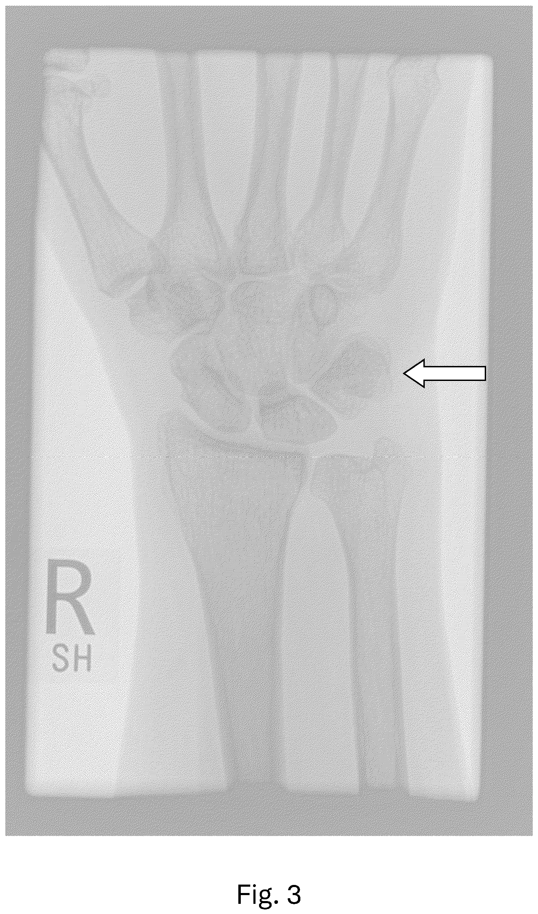

Figure 3 shows a further X-ray image of a right hand, but with different imaging parameters so that any contours or edges of bones are visible in the image with less sharpness. In such images, it seems even harder to recognize whether any bone is fractured. Like in figures 1 and 2, a white arrow indicates in figure 3 where a fracture can be assumed. It may be noted that a neural network may be able to detect a fracture with a higher likelihood than an orthopaedic physician may recognize the same. Nevertheless, in a case as in the example X-ray images a further image from a different imaging direction should be generated so as to assess whether the fracture is in the bone in the front, e.g. the pisiform bone, or in the back, e.g. the triquetrum bone.

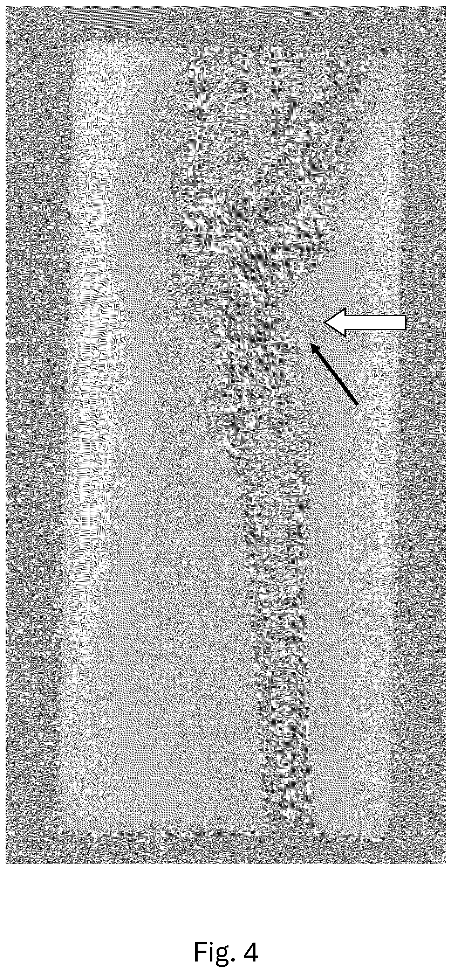

The device according to the present disclosure may provide information to a physician to generate an X-ray image of the hand joint from a side. The result of such imaging may be the X-ray image as in figure 4. The white arrow points to the location at which a fracture can be assumed. The thinner, black arrow points into a gap visible between bone parts. From such a view, a fracture is detectable and the specific location of the fracture parts hints to a fracture of the triquetrum bone.

Summarizing the example of figures 1 to 4, a patient may have an injury of the hand so that an X-ray image of the hand is generated as a top view. The device according to the present disclosure is interpreting the image may be able to recognize a fracture at one of the bones of the hand joint. Due to an uncertainty whether the fracture is on the one or the other of two bones lying in front of each other, the device provides an output indicating that a further X-ray image should be generated as a side view. Based on such a side view, it is possible to determine that the triquetrum bone is fractured.

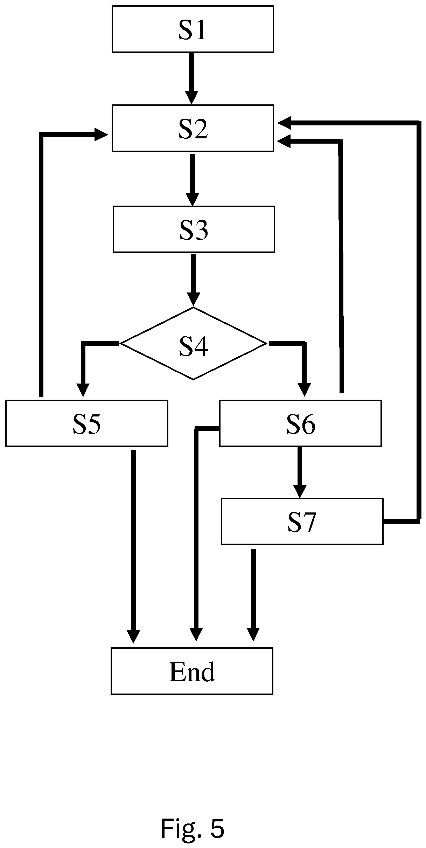

In figure 5, a flow chart is illustrated with steps of a method in accordance with the present disclosure. It will be understood that steps of methods described herein, and in particular of the method described in connection with flow chart, are major steps, wherein these major steps might be differentiated or divided into several sub-steps. Furthermore, additional sub-steps might be between these major steps. It will also be understood that only part of the whole method may constitute the invention, i.e. steps may be omitted or summarized.

In a first step S 1, a physician may examine a patient and may realize that a part of the patient's body is injured. Due to the kind of injury, the question is whether a bone is fractured.

In step S2, image data are received, wherein the image data represent one X-ray image. In other words, an X-ray image is generated showing the injured part of the patient's body.

In step S3, the image data are interpreted by a processing unit analysing the information present in the image data. In particular, it is intended to identify edges and irregularities at the bone structures visible in the X-ray image. The interpretation of the image data may be supported by a neural network and/or an edge detection algorithm. As a result of that step, at least a likelihood of a fracture is estimated.

In step S4, when the likelihood is below a predetermined value, the method continues with step S5, and when the likelihood is above the predetermined value, the method continues with step S6.

In step S5, the device may provide information that the bones in the X-ray image are not fractured, wherein this information may be provided with a sufficient certainty. As a result, the method may come to an end. Alternatively, an output may be provided including information of an imaging direction allowing a further assessment of the injured part of the patient's body, so that the method goes back to step S2 based on an X-ray image generated from the new imaging direction.

In step S6, an output in provided including information of an imaging direction allowing a confirmation of the fracture. Such a new image may be provided, and the method continues with step S2 again.

However, it may also be contemplated that the device is able to recognize the fracture in the X-ray image with sufficient certainty. In such a case the device may classify the fracture in step S7 as a specific fracture out of a plurality of possible fractures. Following that classification, information may be given of an imaging direction allowing a confirmation of the classification of the fracture so that the method continues with step S2 based on a new image.

After detecting and optionally classifying, the method may end with information about the fracture as support for a physician searching for an appropriate therapy of the injured part of the patient.

While examples have been illustrated and described in detail in the figures and afore-going description, such illustrations and descriptions are to be considered illustrative or exemplary and not restrictive, and the invention is not limited to the disclosed examples.

Other variations to the disclosed embodiments can be understood and effected by those skilled in the art in practising the claimed invention, from a study of the figures, the description and the appended claims. In the claims, the word "comprising" does not exclude other elements or steps, and the indefinite article "a" or "an" does not exclude a plurality. A single processor or other unit may fulfil the functions of several items recited in the claims.

The mere fact that certain measures are recited in mutually different dependent claims does not indicate that a combination of these measures can not be used to advantage.

## Claims

1. A device for assisting in fracture diagnosis, the device comprising a processing unit and a computer program product, wherein the computer program product when executed on the processing unit causes the device to
receive image data,
interpret the image data, and
provide an output based on the image data interpretation,
wherein the received image data represents an anatomical region including an injured part of a human body, wherein the image data include data of a single X-ray projection image,
wherein interpreting the image data includes estimating the likelihood of a fracture,
wherein, when the likelihood is below a predetermined value, the provided output includes information of an imaging direction allowing a further assessment of the injured part of the human body, and
wherein, when the likelihood is above the predetermined value, the provided output includes information of an imaging direction allowing a confirmation of the fracture.

2. The device of claim 1, wherein the device is caused, when the likelihood is above the predetermined value, to classify the fracture as a specific fracture out of a plurality of possible fractures, and
wherein the provided output includes information of an imaging direction allowing a confirmation of the classification of the fracture.

3. The device of any one of claims 1 and 2, wherein the interpretation of the image data is based on a neural network being trained based on 3D image data of different fractures of bones and on 2D projections from different directions showing those fractures.

4. The device of any one of claims 1 to 3, wherein the device is caused to determine the imaging direction of the X-ray projection image, and wherein, when the likelihood is above the predetermined value, the output include information regarding a deviation between the determined imaging direction and the imaging direction allowing the confirmation of the fracture.

5. The device of claim 4, wherein the information regarding the deviation is provided to a robotic X-ray imaging device causing the same to generate a new X-ray image from the imaging direction allowing the confirmation of the fracture.

6. The device of any one of claims 1 to 5, wherein the device is caused to assess the feasibility of generating the new X-ray image taking into account movement restrictions for the X-ray imaging device.

7. The device of any one of claims 1 to 6, wherein the predetermined value is between 0,5 and 0,9, preferably between 0,6 and 0,8.

8. The device of any one of claims 1 to 6, wherein the predetermined value is 0,7.

9. A method of assisting in fracture diagnosis, the method comprising the steps of
receiving image data,
interpreting the image data, and
providing an output based on the image data interpretation,
wherein the received image data represents an anatomical region including an injured part of a human body, wherein the image data include data of a single X-ray projection image,
wherein interpreting the image data includes estimating the likelihood of a fracture,
wherein, when the likelihood is below a predetermined value, the provided output includes information of an imaging direction allowing a further assessment of the injured part of the human body, and
wherein, when the likelihood is above the predetermined value, the provided output includes information of an imaging direction allowing a confirmation of the fracture.

10. The method of claim 9, including the step of, when the likelihood is above the predetermined value, classifying the fracture as a specific fracture out of a plurality of possible fractures,
wherein the provided output includes information of an imaging direction allowing a confirmation of the classification of the fracture.

11. The method of any one of claims 9 and 10, including the step of providing the output to a robotic X-ray imaging device causing the same to generate a new X-ray image from the imaging direction allowing the confirmation of the fracture.

12. The method of any one of claims 9 to 11, including the step of assessing the feasibility of generating the new X-ray image taking into account movement restrictions for the X-ray imaging device.

13. The method of any one of claims 9 to 12, wherein the predetermined value is between 0,5 and 0,9, preferably between 0,6 and 0,8.

14. A computer program product including instructions, when executed on a processing unit of a device according to any one of claims 1 to 8, causes the device to perform the method according to any one of claims 9 to 13, respectively.
